# EUROPÄISCHE PATENTSCHRIFT

(11) **EP 2 509 937 B1**
(45) Veröffentlichungstag und Bekanntmachung des Hinweises auf die Patenterteilung: **08.10.2014**
(21) Anmeldenummer: 10798990.7
(22) Anmeldetag: 07.12.2010
(51) Int. Cl.: C07C 231/06, C07C 233/13, C07C 209/62, C07C 211/15

(54) **VERFAHREN ZUR HERSTELLUNG VON 2,2-DIFLUORETHYLAMIN SOWIE SEINER SALZE AUSGEHEND VON DIFLUORACETONITRIL**
METHOD FOR PRODUCING 2,2-DIFLUORETHYLAMINE AND ITS SALTS STARTING FROM DIFLUOROACETONITRILE
PROCÉDÉ DE PRODUCTION DE 2,2-DIFLUORÉTHYLAMINE ET DE SES SELS À PARTIR DE DIFLUORACÉTONITRILE

(30) Priorität: 11.12.2009 EP 09178860; 15.12.2009 US 286607 P
(43) Veröffentlichungstag der Anmeldung: 17.10.2012
(73) Patentinhaber: Bayer Intellectual Property GmbH, 40789 Monheim (DE)
(72) Erfinder: LUI, Norbert, 51519 Odenthal (DE); ANTONS, Stefan, 51373 Leverkusen (DE); MORADI, Wahed, Ahmed, 40789 Monheim (DE)
(86) Internationale Anmeldenummer: PCT/EP2010/069036
(87) Internationale Veröffentlichungsnummer: WO 2011/069994

(56) Entgegenhaltungen:
- US-A- 4 030 994
- WODZINSKA J ET AL: "pKa-dependent formation of amides in water from an acyl phosphate monoester and amines", JOURNAL OF ORGANIC CHEMISTRY 20080620 AMERICAN CHEMICAL SOCIETY US, Bd. 73, Nr. 12, 20. Juni 2008 (2008-06-20) , Seiten 4753-4754, XP002573226,
- DONETTI A ET AL: "N-(fluoroethyl)(imidazolylphenyl)formamid ines. The issue of the active species of mifentidine.", JOURNAL OF MEDICINAL CHEMISTRY MAY 1989, Bd. 32, Nr. 5, Mai 1989 (1989-05), Seiten 957-961, XP002573225, ISSN: 0022-2623 in der Anmeldung erwähnt
- KLUGER R ET AL: "Carboxylic acid participation in amide hydrolysis. Evidence that separation of a nonbonded complex can be rate determining", JOURNAL OF THE AMERICAN CHEMICAL SOCIETY, AMERICAN CHEMICAL SOCIETY, NEW YORK, USA, Bd. 104, Nr. 10, 1. Januar 1982 (1982-01-01), Seiten 2891-2897, XP002504652, ISSN: 0002-7863 in der Anmeldung erwähnt
- DICKEY ET AL: "Fluorinated Aminoanthraquinone dyes", INDUSTRIAL AND ENGENEERING CHEMISTRY,, Bd. 48, Nr. 2, 1. Januar 1956 (1956-01-01) , Seiten 209-213, XP002559402,
- GRUNEWALD G L ET AL: "Application of the Goldilocks effect to the design of potent and selective inhibitors of phenylethanolamine N-methyltransferase: balancing Pka and steric effects in the optimization of 3-methyl-1,2,3,4-tetrahydroisoquinoline inhibitors by beta-fluorination", JOURNAL OF MEDICINAL CHEMISTRY, USAMERICAN CHEMICAL SOCIETY. WASHINGTON, Bd. 49, Nr. 10, 1. Januar 2006 (2006-01-01), Seiten 2939-2952, XP002487200,

## Beschreibung

Die vorliegende Erfindung betrifft ein Verfahren zur Herstellung von 2,2-Difluorethylamin der Formel (I) sowie seinen Salzen wie beispielsweise Sulfate, Hydrochloride oder Acetate, das von Difluoracetonitril ausgcht.

2,2-Difluorethylamine und seine Salze sind wichtige Zwischenprodukte zur Herstellung von Wirkstoffen, insbesondere agrochemischen Wirkstoffen. Es sind verschiedene Herstellungsmethoden für 2,2-Difluorethylamin bekannt.

Donetti et al. (J. Med. Chem. 1989, 32, 957-961) beschreiben beispielsweise die Synthese von 2,2-Difluorethylamin-Hydrochlorid ausgehend von 2,2-Difluoracetamid, in der das entsprechende Amid mit einer Diboran-Lösung in Tetrahydrofuran (THF) reduziert wird. Kluger et al, beschreiben in JACS 1982, 104, 10,2891-2897 die Reduktion von 2,2-Difluoracetamid mit Natriumboranat und Bortrifluoridetherat zum 2,2-Difluorethylamin.

Wodzinska et al., "pKa-dependent formation of amides in water from an acyl phosphate monoester and amines", Journal of organic Chemistry, Vol. 73, no. 12, (2008), pp. 4753 - 4754 beschreibt ein Verfahren zur Herstellung von Amiden durch die Reaktion eines Acylphosphatmonoesters und eines Amines. Das erfindungsgemäße Verfahren, in dem 2,2-Difluorethylamin durch katalytische Hydrierung von Difluoracetonitril zum Difluorethylamid und anschließende Spaltung des Difluorethylamids in Gegenwart einer Säure hergestellt wird, wird aber nicht offenbart.

Dickey at al., "Fluorinated Aminoanthraquinone dyes", Industrial and Engineering Chemistry, Bd. 48. Nr. 2, (1956), 209 - 213 offenbart ein Verfahren zur Herstellung von Difluorethylamin durch nucleophile Substitution. Das erfindungsgemäße Verfahren, in dem 2,2-Difluorethylamin durch katalytische Hydrierung von Difluoracetonitril zum Difluorethylamid und anschließende Spaltung des Difluorethylamids in Gegenwart einer Säure hergestellt wird, wird aber nicht offenbart.

US-A-4,030,994 offenbart ein Verfahren zur Herstellung von Difluorethylamin durch direkte Fluorierung des Ethylamins mit Fluoroxy-trifluoromethan. Das erfindungsgemäße Verfahren, in dem 2,2-Difluorethylamin durch katalytische Hydrierung von Difluoracetonitril zum Difluorethylamid und anschließende Spaltung des Difluorethylamids in Gegenwart einer Säure hergestellt wird, wird aber nicht offenbart.

Auch die in dem erfindungsgemäßen Verfahren als Zwischenprodukte erzeugten Difluorothylamide der Formel (III) werden in keinem der genannten Dokumente offenbart.

Die geringe Ausbeute und der Einsatz von teuren und gefährlichen Chemikalien wie z.B. Natriumboranat/BF₃ oder Diboran verhindern, dass die Verfahren nach Donetti et al. und Kluger et al. für die großtechnische Herstellung von 2,2-Difluorethylamin geeignet sind. Alle diese Verfahren sind unwirtschaftlich und die großtechnische Realisierung ist mit hohen Kosten verbunden.

Ein kostengünstiges Herstellungsverfahren besteht in der Hydrierung von Difluoracetonitril, welches als Ausgangsstoff leicht verfügbar ist. Es lässt sich beispielsweise aus Difluoracetamid herstellen (Swarts et al., Bulletin des Societes Chimiques Belges 1922, 31, 364-5), Grunewald et al., J. Med Chem. 2006, 49 (10), 2939-2952). Die katalytische Hydrierung von Trifluoracetonitril unter Verwendung von PtO₂ ist von Gilman et al. (JACS 1943, 65 (8), 1458 - 1460) beschrieben, wobei Trifluorethylamin-Hydrochlorid erhalten wird.

Die Erfinder haben nun festgestellt, dass das von Gilman et al. für Trifluoracetonitril beschriebene Verfahren sich nicht für die Hydrierung von Difluoracetonitril eignet. Führt man die Hydrierung von Difluoracetonitril unter den beschriebenen Bedingungen durch, so wird 2,2-Difluorethylamin nur in Spuren erhalten, während ansonsten eine Vielzahl höheralkylierte Reaktionsprodukte erhalten werden.

Des Weiteren wurde festgestellt, dass man bei der katalytischen Hydrierung von Difluoracetonitril in reinem Eisessig oder in Toluol zwar Difluorethylamin erhält, allerdings waren die Umsetzungen nicht selektiv und das Produkt konnte aufgrund des niedrigen Siedepunktes aus der Reaktionsmischung nicht isoliert werden.

Es stellt sich daher die Aufgabe, ein Verfahren bereitzustellen, mit dem Difluoracetonitril zu 2,2-Difluorethylamin selektiv und in guten Ausbeuten umgesetzt werden kann. Es wurde nun gefunden, dass man 2,2-Difluorethylamin der Formel (1) erhalten kann, indem man zunächst Difluoracetonitril der Formel (II) in einem ersten Schritt durch katalytische Hydrierung zum N(2,2-Difluorethyl)amid der Formel (III) reduziert und anschließend das so erhaltene N(2,2-Difluorethyl)amid durch Behandlung mit Säure zum 2,2-Difluorethylamin umsetzt. Die Reaktion ist im nachfolgenden Reaktionsschema dargestellt, wobei R¹ die weiter unten genannten Bedeutungen haben kann.

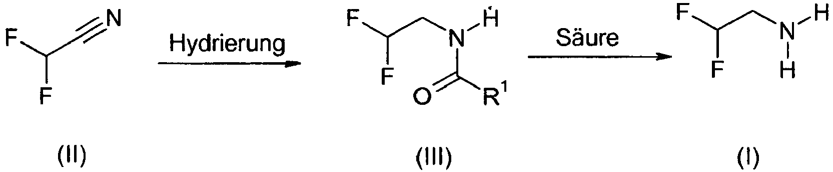

Die vorliegende Erfindung betrifft somit ein Verfahren zur Herstellung von 2,2-Difluorethylamin der Formel (I) umfassend die folgenden Reaktionsschritte
(a) katalytische Hydrierung von Difluoracetonitril der Formel (II) zum entsprechenden Amid der Formel (III) 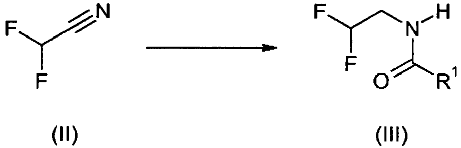 wobei
   R¹ für H, C₁₋₁₂-Alkyl, C₃₋₈-Cycloalkyl, C₁₋₁₂-Haloalkyl, Aryl (z.B. Phenyl), C₁₋₁₂-Alkyl-C₆-lo-aryl steht, R¹ steht bevorzugt für H, Methyl, Trifluormethyl, Ethyl, n-Propyl, iso-Propyl, n-Butyl, iso-Butyl, sec-Butyl,- und t-Butyl, n-Pentyl, n-Hexyl, 1,3-Dimethylbutyl, 3,3-Dimethylbutyl, n-Heptyl, n-Nonyl, n-Decyl, n-Undecyl, n-Dodecyl, Phenyl oder Benzyl, besonders bevorzugt für H, Methyl, t-Butyl oder Phenyl.
   in Gegenwart einer organischen Säure der allgemeinen Formel (IVa), eines Säurechlorids der allgemeinen Formel (IVb) oder Säureanhydrids der allgemeinen Formel (IVc) 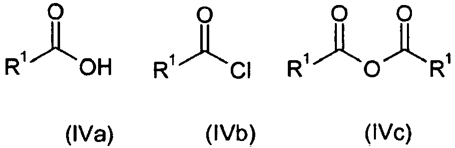 oder Mischungen davon, wobei R¹ die vorgenannte Bedeutung hat, bevorzugt in Gegenwart von CF₃COOH, CH₃COOH, CH₃COCl, Benzoylchlorid, Essigsäureanhydrid, Pivaloylsäureanhydrid, t-Butylessigsäureanhydrid, Trifluoressigsäureanhydrid oder Benzoylanhydrid oder Mischungen davon, besonders bevorzugt in Gegenwart von CH₃COOH, CH₃COCl oder Essigsäureanhydrid oder Mischungen davon; und
   (b) Umsetzung des Difluorethylamids der Formel (III) zu 2,2-Difluorethylamin der Formel (I) durch Zugabe einer Säure, die geeignet ist, das Difluorethylamid zu spalten

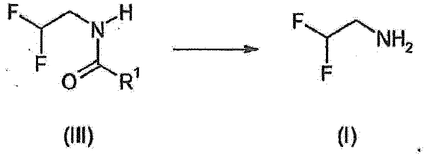

Die Erfindung betrifft weiterhin die durch das erfindungsgemäße Verfahren gewonnene Difluorethylamid-Zwischenstufe der allgemeinen Formel (III), wobei R¹ für H, C₁₋₁₂ Alkyl, C₂₋₈-Cycloalkyl, C₁₋₁₂-Haloalkyl, C₁₋₁₂-Alkyl-C₆₋₁₀-aryl steht. Die erfindungsgemäße katalytische Hydrierung im Schritt (a) findet an Gegenwart eines Katalysators statt, wobei gasförmiger Wasserstoff in das Reaktionsgefäß eingeleitet oder im Reaktionsgefäß *in situ* durch die Verwendung von Ameisensäure oder Hydrazin sowie deren Derivate oder Salze davon, erzeugt wird.

Für die erfindungsgemäße katalytische Hydrierung im Reaktionsschritt (a) kann als Katalysator jeder dem Fachmann bekannte, zur katalytischen Hydrierung geeignete Katalysator verwendet werden. In Frage kommen beispielsweise Palladiumkatalysatoren, Platinkatalysatoren, Raney-Nickel-Katalysatoren, Lindlar-Katalysatoren, Ruthenium- und Rhodiumkatalysatoren. Neben diesen heterogenen Katalysatoren können auch homogene Katalysatoren verwendet werden. Geeignete Katalysatoren enthalten bevorzugt ein oder mehrere Metalle der Gruppen 8 - 10 des Periodensystems, insbesondere ein oder mehrere Metalle ausgewählt unter Eisen, Ruthenium, Osmium, Kobalt, Rhodium, Iridium, Nickel, Palladium und Platin. Die Metalle können in jedweder chemischen Form vorliegen, z.B. elementar, kolloidal, als Salze oder Oxide, zusammen mit Komplexbildner als Chelate, oder als Legierungen, wobei die Legierungen neben den oben angeführten Metalle auch andere Metalle, wie beispielsweise Aluminium beinhalten können. Die Metalle können in geträgerter Form, d.h. auf einen beliebigen, vorzugsweise anorganischen, Träger aufgebracht, vorliegen. Als Träger eigenen sich beispielsweise Kohlenstoff (Kohle oder Aktivkohle), Aluminiumoxid, Siliciumdioxid, Zirkondioxid oder Titandioxid. Erfindungsgemäß bevorzugte Katalysatoren enthalten ein oder mehrere Metalle der Gruppen 8 - 10 des Periodensystems auf einem anorganischen Träger. Erfindungsgemäß besonders bevorzugt sind Katalysatoren, die Platin und/oder Palladium beinhalten und gegebenenfalls auf einem anorganischen Träger aufgebracht sind. Solche Katalysatoren sind beispielsweise PtO₂, Pd(OH)₂ auf Aktivkohle (Pearlman Katalysator), Raney-Nickel, und Lindlar-Katalysatoren.

Im erfindungsgemäßen Verfahren wird der Katalysator, bezogen auf das eingesetzte Difluoracetonitril, in einer Konzentration von etwa 0,01 bis etwa 30 Gew.-% verwendet. Bevorzugt wird der Katalysator in einer Konzentration von etwa 0,1 bis etwa 12 Gew.-% verwendet, besonders bevorzugt von etwa 0,1 bis etwa 2 Gew.-%.

In Schritt (a) des erfindungsgemäßen Verfahrens wird gewöhnlicherweise in einem ersten Schritt (i) Difluoracetonitril und der Katalysator mit der organischen Säure, Säurechlorid, Säureanhydrid oder Mischungen davon vorgelegt und in einem zweiten Schritt (ii) Wasserstoff eingeleitet oder *in situ* erzeugt. Die Umkehrung der Schritte (i) und (ii) ist möglich. Auch kann kontinuierlich oder diskontinuierlich (schub- bzw. batchweise) hydriert werden.

Die katalytische Hydrierung kann unter Überdruck (d.h. bis zu etwa 200 bar) in einem Autoklaven oder bei Normaldruck in einer Wasserstoffgasatmosphäre durchgeführt werden. Insbesondere bei hohen Reaktionstemperaturen kann es hilfreich sein, bei erhöhtem Druck zu arbeiten. Die (zusätzliche) Druckerhöhung kann durch Zuleiten eines Inertgases, wie Stickstoff oder Argon, bewirkt werden. Die erfindungsgemäße Hydrierung erfolgt bevorzugt bei einem Druck im Bereich von etwa 1 bis etwa 100 bar, besonders bevorzugt bei einem Druck im Bereich von etwa 5 bis etwa 25 bar.

Die im Reaktionsschritt (a) vorhandene organische Säure, Säurechlorid oder Säureanhydrid oder Mischungen davon führen dazu, dass das gebildete Difluorethylamin dem Hydrierprozess entzogen wird und nicht zum (CF₂HCH₂)₂NH abreagiert.

Die notwendige Menge der im Reaktionsschritt (a) bezogen auf Difluoracetonitril vorhandene organische Säure, Säurechlorid oder Säureanhydrid kann vom Fachmann einfach durch Routineversuche ermittelt werden. Das molare Verhältnis von Difluoracetonitril zur eingesetzten organischen Säure, Säurechlorid oder Säureanhydrid oder Mischungen davon kann beispielsweise etwa 0,5 bis 10, oder etwa 0,9 bis 2 betragen. Ein Verhältnis von etwa 1 bis 1,1 ist bevorzugt. Der Einsatz größerer Mengen an organischer Säure, Säurechlorid oder Säureanhydrid oder Mischungen davon ist grundsätzlich möglich, ist aber aus wirtschaftlichen Gründen nachteilig.

Bevorzugte Reaktionstemperaturen für die Hydrierung im Reaktionsschritt (a) reichen von -20 °C bis 100 °C, wobei Temperaturen von 0 °C bis 40 °C bevorzugt sind.

Die Reaktionsdauer der Hydrierung beträgt im Allgemeinen 30 Minuten bis 24 Stunden, wobei kürzere oder längere Reaktionszeiten sich nicht nachteilig auswirken.

Im erfindungsgemäßen Reaktionsschritt (b) wird das Amid der Formel (III) mit einer geeigneten Säure zum 2,2-Difluoramin umgesetzt.

Nach Reaktionsschritt (a) kann das Amid der Formel (III) auch durch Entfernen des Katalysators und des Lösungsmittels, sofern vorhanden, isoliert und Reaktionsschritt (b) zugeführt werden.

Die im Reaktionsschritt (b) verwendbaren Säuren sind ausgewählt unter Phosphorsäure (H₃PO₄), Schwefelsäure (H₂SO₄), Salzsäure (HCl), Bromwasserstoffsäure (HBr), Flusssäure (HF), Kaliumhydrogensulfat (KHSO₄), CF₃COOH, CF₃SO₃H, CH₃COOH, und p-Tolylsulfonsäure.

Bevorzugte Reaktionstemperaturen für die Spaltung des Difluoramids der Formel (III) im Reaktionsschritt (b) reichen von etwa 0 °C bis etwa 100 °C.

Es ist im Allgemeinen vorteilhaft, das erfindungsgemäße Verfahren in Gegenwart von Lösungsmitteln (Verdünnungsmitteln) durchzuführen. Die katalytische Hydrierung kann jedoch auch ohne ein Lösungsmittel durchgeführt werden. Lösungsmittel werden vorteilhafterweise in einer solchen Menge eingesetzt, dass das Reaktionsgemisch während des ganzen Verfahrens gut rührbar bleibt. Vorteilhafterweise wird, bezogen auf das eingesetzte Difluoracetonitril, die 1 bis 50-fache Lösungsmittelmenge, bevorzugt die 2 bis 40-fache Lösungsmittelmenge, besonders bevorzugt die 2 bis 30-fache Lösungsmittelmenge verwendet.

Als Lösungsmittel zur Durchführung des erfindungsgemäßen Verfahrens kommen alle unter den Reaktionsbedingungen inerten organischen Lösungsmittel in Frage, wobei die Art des verwendeten Lösungsmittels von der Art der Reaktionsdurchführung, insbesondere von der Art des verwendeten Katalysators und/oder der Wasserstoffquelle (Einleiten von gasförmigen Wasserstoff oder *in situ* Erzeugung) abhängt. Unter Lösungsmittel werden erfindungsgemäß auch Gemische reiner Lösungsmittel verstanden.

Erfindungsgemäß geeignete Lösungsmittel sind insbesondere Ether, wie Ethylpropylether, n-Butylether, Anisol, Phenetol, Cyclohexylmethylether, Dimethylether, Diethylether, Dimethylglycol Diphenylether, Diproplether, Diisopropylether, Di-n-butylether, Diisobutylether, Diisoamylether, Ethylenglycoldimethylether, Isopropylethylether, Methyl-tert-butylether, Tetrahydrofuran, Methyl-Tetrahydrofuran, Dioxan, Dichlordiethylether und Polyether des Ethylenoxids und/oder Propylenoxids; aliphatische, cycloaliphatische oder aromatische Kohlenwasserstoffe wie Pentan, Hexan, Heptan, Oktan, Nonan und technische Kohlenwasserstoffe welche durch Fluor- und Chloratome substituiert sein können, wie Methylenchlorid, Dichlormethan, Trichlormethan, Tetrachlorkohlenstoff, Fluorbenzol, Chlorbenzol oder Dichlorbenzol; beispielsweise sogenannte White Spirits mit Komponenten mit Siedepunkten im Bereich beispielsweise von 40 °C bis 250 °C, Cymol, Benzinfraktionen innerhalb eines Siedeinterwalles von 70 °C bis 190 °C, Cyclohexan, Methylcyclohexan, Petrolether, Ligroin, Octan, Benzol, Toluol, Chlorbenzol, Brombenzol, Xylol; Ester wie Methyl-, Ethyl-, Butyl-, Isobutylacetat, sowie Dimethyl-, Dibutyl- oder Ethylencarbonat. Organische Säuren, wie Ameisensäure oder Essigsäure. Als erfindungsgemäßes Lösungsmittel kann auch Wasser verwendet werden. Im Reaktionsschritt (a) kann auch die in der Reaktion vorhandene organische Säure, das Säurechlorid oder Anhydrid sowie Mischungen davon als Lösungsmittel verwendet werden.

Erfindungsgemäß bevorzugte Lösungsmittel sind im Reaktionsschritt (a) Toluol, Tetrahydrofuran, Methyl-Tetrahydrofuran oder Mischungen davon.

Im Reaktionsschritt (b) ist Wasser als erfindungsgemäßes Lösungsmittel bevorzugt.

Die Aufarbeitung und Reinigung kann über das freie Amin oder über dessen Salze erfolgen. Liegt nach dem erfindungsgemäßen Verfahren das 2,2-Difluorethylamin frei vor, so wird es, wenn nötig, durch Destillation gereinigt. Liegt 2,2-Difluorethylamin als Salz vor, dann erfolgt die Reinigung, wenn nötig, bevorzugt durch Kristallisation. Bevorzugte Salze sind zum Beispiel Sulfate, Hydrochloride oder Acetate.

Wasserlösliche Salze von 2,2-Difluorethylamin werden im Allgemeinen durch Extraktion aus einer wässrigen Lösung gereinigt. Das freie 2,2-Difluorethylamin wird durch Umsetzen des entsprechenden Salzes mit organischen oder anorganischen Basen (z.B. NaHCO₃, Na₂CO₃ oder NaOH) freigesetzt. Anschließend wird das Difluorethylamin direkt aus der wässrigen Lösung abdestilliert oder in ein organisches Lösungsmittel extrahiert.

Die vorliegende Erfindung wird anhand der nachfolgenden Beispiele näher erläutert, wobei die Beispiele nicht in die Erfindung einschränkender Weise zu interpretieren sind.

### Herstellungsbeispiele:

### Synthese von N-(2,2-Difluorethyl)acetamid

20 g (0,259 mol) Difluoracetonitril und 26,5 g (0,259 mol) Essigsäureanhydrid werden in 242 ml Tetrahydrofuran gelöst und über 0,66 g (0,31 mmol) Palladium auf Aktivkohle (5% Pd) mit 50 bar Wasserstoff bis zur Druckkonstanz hydriert. Der Autoklav wird gekühlt, sodass die Reaktionstemperatur nicht über 20 °C steigt. Die Reaktionsmischung wird über Kieselgur abfiltriert. Nach Entfernen des Lösungsmittels erhält man 34.9 g (75,5% GC-MS-Reinheit) N-(2,2-Difluorethyl)acetamid.

¹H-NMR (400 MHz, d₆-DMSO): 8,24 (1H, sb, NH), 5,98 (1H, dt, ³J_{HF} = 60 Hz; ³J_{HH} = 3,9 Hz), 3,56-3,49 (2H, m), 1,87 (3H, s).

¹³C-NMR (600 MHz, d₆-THF): 171,7 (CO), 115,3 (CHF₂), 42,5 (CH₂), 22,3 (CH₃).

¹⁹F-NMR (376 MHz, D₂O, CFCl₃ interner Standard): -121,3 (dt, ²J_{FH} = 56,1 Hz; ³J_{FH} = 16,1 Hz).

### Synthese von 2,2-Difluorethylaminhydrochlorid

10 g (81,23 mmol) N-(2,2-Difluorethyl)acetamid werden in 16 g Wasser vorgelegt und mit 18,5 g (162,5 mmol, 32%ig) Salzsäure versetzt. Die Reaktionsmischung wird eine Stunde bei 90 °C gerührt, auf Raumtemperatur abgekühlt und anschließend das Lösungsmittel entfernt. Der Rückstand wird mit Toluol azeotropiert. Man erhält 8,70 g 2,2-Difluorethylaminhydrochlorid (91,1 % Ausbeute bezogen auf N-(2,2-Difluorethyl)acetamid).

¹H-NMR (400 MHz, D₂O): 6,31 (1H, dt, ³J_{HF} = 53,34 Hz; ³J_{HH} = 2,6 Hz), 3,52 (2H, dt, ³J_{HF} = 16,32 Hz; ³J_{HH} = 2,6 Hz).

## Patentansprüche

1. Verfahren zur Herstellung von 2,2-Difluorethylamin der Formel (I), umfassend die folgenden Reaktionsschritte
(a) katalytische Hydrierung von Difluoracetonitril der Formel (II) zum Difluorethylamid der Formel (III) 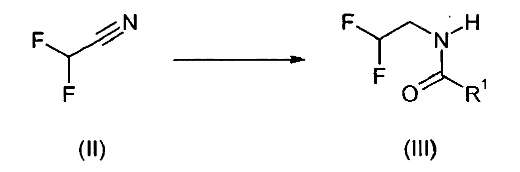 wobei
R¹ für H, C₁₋₁₂-Alkyl, C₃₋₈-Cycloalkyl, C₁₋₁₂-Haloalkyl, Aryl, C₁₋₁₂-Alkyl-C₆₋₁₀-aryl steht,
in Gegenwart einer organischen Säure der allgemeinen Formel (IVa), eines Säurechlorids der allgemeinen Formel (IVb) oder Säureanhydrids der allgemeinen Formel (IVc) oder Mischungen davon 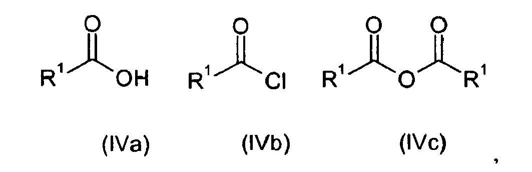 wobei R¹ die vorgenannte Bedeutung hat; und
(b) Umsetzung des Difluorethylamids der Formel (III) zu 2,2-Difluorethylamin durch Zugabe einer Säure, die geeignet ist, das Difluorethylamid zu spalten

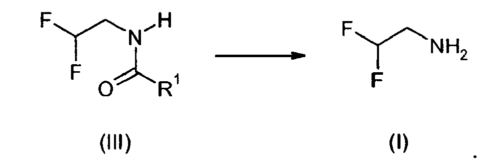

2. Verfahren nach Anspruch 1, wobei R¹ für H, Methyl, Trifluonnethyl, Ethyl, n-Propyl, iso-Propyl, n-Butyl, iso-Butyl, sec-Butyl,- und t-Butyl, n-Pentyl, n-Hexyl, 1,3-Dimethylbutyl, 3,3-Dimethylbutyl, n-Heptyl, n-Nonyl, n-Decyl, n-Undecyl, n-Dodecyl, Phenyl oder Benzyl steht.

3. Verfahren nach Anspruch 1 oder 2, wobei die im Reaktionsschritt (a) vorhandene Säure in einem molaren Verhältnis von Difluoracetonitril zur Säure von 0,5 bis 10 vorliegt.

4. Verfahren nach einem der Ansprüche 1 bis 3, wobei die Verfahrensschritte (a) und (b) ohne Isolierung von Difluorethylamid der Formel (III) durchgeführt werden.

5. Verfahren nach einem der Ansprüche 1 bis 4, wobei der in der katalytischen Hydrierung des Reaktionsschrittes (a) eingesetzte Katalysator Palladium, Platin, Raney-Nickel oder Rhodium enthält.

6. Verfahren nach einem der Ansprüche 1 bis 5, wobei bei katalytischer Hydrierung gasförmiger Wasserstoff in das Reaktionsgefäß eingeleitet oder *in situ* erzeugt wird.

7. Verfahren nach einem der Ansprüche 1 bis 6, wobei die im Reaktionsschritt (b) zuzugebende Säure, die geeignet ist, das Difluorethylamid zu spalten, ausgewählt ist unter H₃PO₄, H₂SO₄, HCl, HBr, HF, KHSO₄, CF₃COOH, CF₃SO₃H, CH₃COOH und p-Tolylsulfonsäure.

8. Difluorethylamid der Formel (III) zur Verwendung in dem Verfahren wie in einem der Ansprüche 1 bis 7 definiert 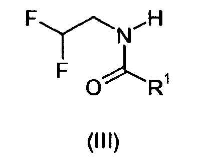 worin
R¹ zur H, C₁₋₁₂-Alkyl, C₃₋₈-Cycloalkyl, C₁₋₁₂-Haloalkyl, C₁₋₁₂-Alkyl-C₆₋₁₀-aryl steht,

9. Difluorethylamid der Formel (III) gemäß Anspruch 8, worin
R¹ für H, Methyl, Trifluormethyl, Ethyl, n-Propyl, iso-Propyl, n-Butyl, iso-Butyl, sec-Butyl,- und t-Butyl, n-Pentyl, n-Hexyl, 1,3-Dimethylbutyl, 3,3-Dimethylbutyl, n-Heptyl, n-Nonyl, n-Decyl, n-Undecyl, n-Dodecyl, oder Benzyl steht.

## Claims

1. Process for preparing 2,2-difluoroethylamine of the formula (I), comprising the following reaction steps:
(a) catalytically hydrogenating difluoroacetonitrile of the formula (II) to the difluoroethylamide of the formula (III) 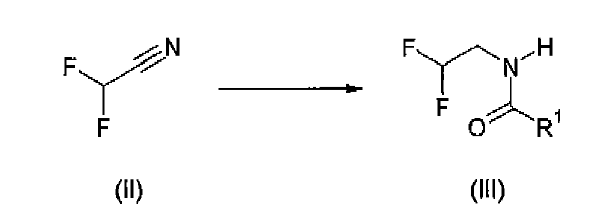 where
R¹ is H, C₁₋₁₂-alkyl, C₃₋₈-cycloalkyl, C₁₋₁₂-haloalkyl, aryl, C₁₋₁₂-alkyl-C₆-₁₀-aryl,
in the presence of an organic acid of the general formula (IVa), of an acid chloride of the general formula (IVb) or of an acid anhydride of the general formula (IVc), or a mixture thereof, 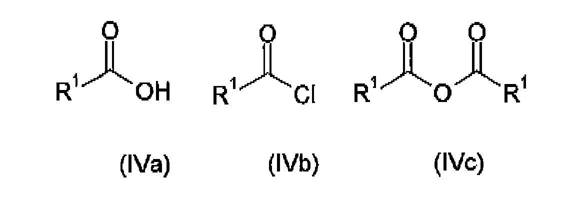 where R¹ is as defined above; and
(b) converting the difluoroethylamide of the formula (III) to 2,2-difluoroethylamine by adding an acid which is suitable for cleaving the difluoroethylamide

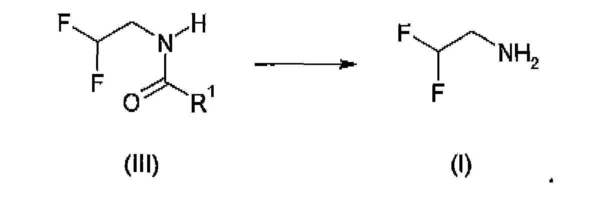

2. Process according to Claim 1, wherein R¹ is H, methyl, trifluoromethyl, ethyl, n-propyl, isopropyl, n--butyl, isobutyl, sec-butyl, and t-butyl, n-pentyl, n-hexyl, 1,3-dimethylbutyl, 3,3-dimethylbutyl, n-heptyl, n-nonyl, n-decyl, n-undecyl, n-dadecyl, phenol or benzyl.

3. Process according to Claim 1 or 2, wherein the acid present in reaction step (a) is present in a molar ratio of difluoroacetonitrile to the acid of 0.5 to 10.

4. Process according to any of Claims 1 to 3, wherein process steps (a) and (b) are performed without isolation of difluoroethylamide of the formula (III).

5. Process according to any of Claims 1 to 4, wherein the catalyst used in the catalytic hydrogenation of reaction step (a) comprises palladium, platinum, Raney nickel or rhodium.

6. Process according to any of Claims 1 to 5, wherein catalytic hydrogenation involves introducing gaseous hydrogen into the reaction vessel or producing it *in situ.*

7. Process according to any of Claims 1 to 6, wherein the acid which is suitable for cleaving the difluoroethylamide and is to be added in reaction step (b) is selected from H₃PO₄, H₂SO₄, HCl, HBr, HF, KHSO₄, CF₃COOH, CF₃SO₃H, CH₃COOH and p-toluenesulphonic acid.

8. Difluoroethylamide of the formula (III) for use in the process as defined in any of Claims 1 to 7 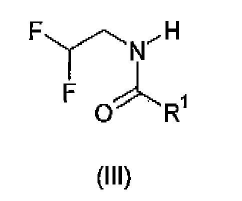 in which
R¹ is H, C₁₋₁₂-alkyl, C₃₋₈-cycloalkyl, C₁₋₁₂-haloalkyl, C₁₋₁₂-alkyl-C₆₋₁₀-aryl.

9. Difluoroethylamide of the formula (III) according to Claim 8, in which
R¹ is H, methyl, trifluoromethyl, ethyl, n-propyl, isopropyl, n-butyl, isobutyl, sec-butyl, and t-butyl, n-pentyl, n-hexyl, 1,3-dimethylbutyl, 3,3-dimethylbutyl, n-heptyl, n-nonyl, n-decyl, n-undecyl, n-dodecyl, or benzyl.

## Revendications

1. Procédé pour la préparation de 2,2-difluoroéthylamine de formule (I), comprenant les étapes de réaction suivantes
(a) hydrogénation catalytique de difluoroacétonitrile de formule (II) en difluoroéthylamide de formule (III) 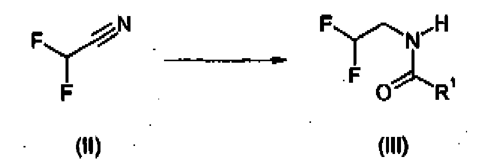 où
R¹ représente H, C₁₋₁₂-alkyle, C₃₋₈-cycloalkyle, C₁₋₁₂-halogénoalkyle, aryle, C₁₋₁₂-allkyl-C₆₋₁₀-aryle,
en présence d'un acide organique de formule générale (IVa), d'un chlorure d'acide de formule générale (IVb) ou d'un anhydride d'acide de formule générale (IVc) ou leurs mélanges 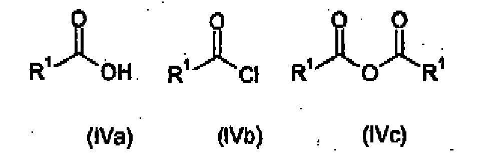 où R¹ a la signification susmentionnée ; et
(b) transformation du difluoroéthylamide de formule (III) en 2,2-difluoréthylamine par addition d'un acide qui convient pour dissocier le difluoroéthylamide

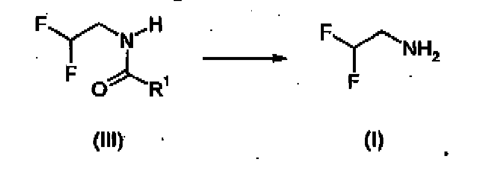

2. Procédé selon la revendication 1, R¹ représentant H, méthyle, trifluorométhyle, éthyle, n-propyle, isopropyle, n-butyle, isobutyle, sec-butyle et t-butyle, n-pentyle, n-hexyle, 1,3-diméthylbutyle, 3,3-diméthylbutyle, n-heptyle, n-nonyle, n-décyle, n-undécyle, n-dodécyle, phényle ou benzoyle.

3. Procédé selon la revendication 1 ou 2, l'acide présent dans l'étape de réaction (a) se trouvant dans un rapport molaire de difluoroacétonitrile à acide de 0,5 à 10.

4. Procédé selon l'une quelconque des revendications 1 3, les étapes de procédé (a) et (b) étant réalisées sans isolement du difluoroéthylamide de formule (III).

5. Procédé selon l'une quelconque des revendications 1 à 4, le catalyseur utilisé dans l'hydrogénation catalytique de l'étape de réaction (a) contenant du palladium, du platine, du nickel de Raney ou du rhodium.

6. Procédé selon l'une quelconque des revendications 1 à 5, de l'hydrogène gazeux étant introduit dans le récipient de réaction ou généré in situ lors de l'hydrogénation catalytique.

7. Procédé selon l'une quelconque des revendications 1 à 6, l'acide ajouté dans l'étape de réaction (b), approprié pour dissocier le difluoroéthylamide, étant choisi parmi H₃PO₄, H₂SO₄, HCl, HBr, HF, KHSO₄, CF₃COOH, CF₃SO₃H, CH₃COOH et l'acide p-toluylsulfonique.

8. Difluoroéthylamide de formule (III) destiné à être utilisé dans le procédé tel que défini dans l'une quelconque des revendications 1 à 7 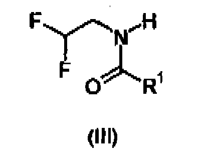 où
R¹ représente H, C₁₋₁₂-alkyle, C₃₋₈-cycloalkyle, C₁₋₁₂-halogénoalkyle, C₁₋₁₂-alkyl-C₆₋₁₀-aryle.

9. Difluoroéthylamide de formule (III) selon la revendication 8, où
R¹ représente H, méthyle, trifluorométhyle, éthyle, n-propyle, isopropyle, n-butyle, isobutyle, sec-butyle et t-butyle, n-pentyle, n-hexyle, 1,3-diméthylbutyle, 3,3-diméthylbutyle, n-heptyle, n-nonyle, n-décyle, n-undécyle, n-dodécyle ou benzyle.
